# NOUVEAU FASCICULE DE BREVET EUROPEEN

(11) **EP 1 351 694 B2**
(45) Date de publication et mention de la décision concernant l'opposition: **27.07.2011**
(45) Mention de la délivrance du brevet: 12.09.2007
(21) Numéro de dépôt: 02700343.3
(22) Date de dépôt: 21.01.2002
(51) Int. Cl.: A61K 31/78, A61K 31/715, A61P 17/02

(54) **FAVORISATION DE LA RECONSTRUCTION CELLULAIRE ET/OU DE LA DIFFERENCIATION CELLULAIRE AVEC SUCRE NON METABOLISABLE ET UN ABSORBANT POLYMERIQUE**
BEGÜNSTIGUNG DER ZELLWIEDERHERSTELLUNG UND/ODER DER ZELLDIFFERENZIERUNG MIT EINEM NICHT METABOLISIERBAREN ZUCKER UND EINEM POLYMERISCHEN ABSORBIERMITTEL
PROMOTING CELL REGENERATION AND/OR CELL DIFFERENTIATION WITH NON-METABOLIZABLE SUGAR AND A POLYMERIC ABSORBENT

(30) Priorité: 19.01.2001 FR 0100738
(43) Date de publication de la demande: 15.10.2003
(73) Titulaire: UENI MEDICA, 29940 La Foret Fouesnant (FR)
(72) Inventeur: JEAN, Daniel, F-63270 Vic-Le-Comte (FR); CARIEL, Léon, F-75006 Paris (FR); SCHWAAB, Veronique, 63670 La Roche Blanche (FR)
(74) Mandataire: Warcoin, Jacques
(86) Numéro de dépôt international: PCT/FR2002/000228
(87) Numéro de publication internationale: WO 2002/056894

(56) Documents cités:
- EP-A- 0 745 392
- WO-A-95/09626
- WO-A-98/48774
- WO-A1-90/11069
- FR-A- 2 243 678
- FR-A- 2 736 835
- CHEMICAL ABSTRACTS, vol. 130, no. 6, 8 février 1999 (1999-02-08) Columbus, Ohio, US; abstract no. 71550, KUSUMOTO MITSUTOSHI E.A.: "Antimicrobial topical compositions for the treatment of wounds" XP002182524 & JP 10 338638 A (TOA YAKUHIN) 22 décembre 1998 (1998-12-22)
- CHEMICAL ABSTRACTS, vol. 132, no. 10, 6 mars 2000 (2000-03-06) Columbus, Ohio, US; abstract no. 127757, HIRATA KENJI E. A.: "Stable povidone-iodine ointments containing polysaccharides for treatment of bedsore and skin injury" XP002182525 & JP 2000 038342 A (KYOWA YAKUHIN KOGYO) 8 février 2000 (2000-02-08)
- DATABASE WPI Week 198939 Derwent Publications Ltd., London, GB; AN 1989-280854 XP002199827 TAKEDA: "Drugs acting on osteoporosis-comprising 2-(4-alkoxy-pyridyl-2-methosulphoxy)-benim idazole" & JP 01 203325 A (TAKEDA), 1989

## Description

La présente invention concerne l'utilisation d'une association telle que définie dans la revendication 1 pour la préparation d'une composition, notamment médicamenteuse, pour favoriser la reconstruction cellulaire et/ou la différenciation cellulaire, en particulier pour favoriser la cicatrisation, avantageusement des escarres.

Il est connu dans l'art antérieur que l'on peut utiliser des sucres dans le traitement des escarres en particulier pour lutter contre les infections bactériennes. On trouve également indiqué que des polymères hydrophiles peuvent être utilisés pour traiter les escarres dans le but d'absorber les exsudats.

L'état de la technique présenté ci-dessous est extrait du livre « L'escarre - évaluation et prise en charge » coordonné par B. BARROIS, D. COLIN, S. DESJOBERT et publié aux éditions FRISON-ROCHE.

Les escarres sont des zones localisées de nécroses tissulaires dues à une ischémie des tissus sous-cutanés. La cause principale du développement des escarres est une compression des tissus mous entre une proéminence osseuse et une surface externe pendant une durée prolongée.

De manière générale, le développement des escarres est expliqué par la pression prolongée de ladite surface externe sur la peau et les tissus sous-jacents et aussi par les phénomènes d'hypoxie ou d'anoxie tissulaire qui en résultent.

Néanmoins, il a été constaté qu'il existe une grande hétérogénéité clinique et que, chez certains patients tels que les personnes âgées, les blessés médullaires ou les malades comateux, la survenue d'escarres ne peut être considérée comme étant l'unique résultat de la conjonction d'un défaut de soins et d'une augmentation de la pression tissulaire. Chez certains malades, en particulier en service de réanimation, une apparition d'escarres localisées dans une zone qui n'est pas un point de compression est souvent constatée.

Outre la compression, le rôle d'autres facteurs intrinsèques est aussi connu. Il s'agit classiquement du cisaillement, de la macération, des infections et du déficit nutritionnel.

Le cisaillement est observé essentiellement chez des patients qui restent en position semi-assise durant une période prolongée, il est engendré par la conjonction d'une hypertension et d'une force tangentielle liée au glissement.

La macération est souvent la conséquence d'une hyperthermie provoquant transpiration et perspiration accrues, qui peut être accompagnée d'incontinence sphinctérienne.

Les infections ont également un rôle d'autant plus déterminant que le patient peut être l'objet d'un déficit immunitaire.

Le déficit nutritionnel se manifeste dans le renouvellement protidique tissulaire, du ravitaillement des tissus en substrats énergétiques, de la carence en vitamines ou en oligo-éléments. La survenue d'une cachexie endogène et en particulier d'un diabète ne peut qu'aggraver cette situation pré-existante.

Différents produits et techniques ont été proposés pour traiter les escarres, les principaux sont présentés ci-dessous.

Les films-pansements qui sont des minces feuilles transparentes souples et extensibles réservées aux escarres non exsudatives.

Les pansements gras faits de tulle ou de gaze imprégné de vaseline et qui peuvent en outre comprendre un antibiotique, un antiseptique ou un corticoïde. L'inconvénient majeur de ces pansements gras est qu'ils contrôlent mal l'exsudat.

Les hydrocolloïdes, au contraire, absorbent l'exsudat en se gélifiant et entretiennent un milieu humide. Le problème posé par ces produits est qu'ils se délitent au contact des exsudats en produisant une odeur nauséabonde.

Les alginates ont un grand pouvoir d'absorption, ils sont utiles lorsque la plaie est très exsudative.

Les hydrogels sont moins absorbants que les alginates, mais leur pouvoir d'humification est très utile dans la phase de détersion des zones de nécrose qui précède la phase de cicatrisation. Ils présentent le désavantage d'avoir un coût élevé.

Les hydrocellulaires sont très absorbants et contrôlent l'exsudat sans le déliter, leur seule indication est le bourgeonnement.

L'application de sucre sur les escarres pour absorber les exsudats a aussi été utilisée à titre de traitement. Cette pratique peut cependant présenter des inconvénients, en particulier lorsque le patient est diabétique car, outre le fait d'augmenter la glycémie du malade, il s'est avéré que, dans ce cas, la cicatrisation est altérée. Adjoindre du sucre sur ces plaies aurait pour conséquence d'aggraver cette altération de la cicatrisation.

D'une façon surprenante, on a pu montrer dans des tests in vitro de cultures cellulaires que l'association comprenant au moins un absorbant et au moins un composé choisi dans le groupe formé par les sucres non métabolisables et les polyols permettait de façon très forte la différenciation cellulaire et la reconstruction tissulaire sans présenter les inconvénients suscités.

La présente invention concerne donc l'utilisation d'une association telle que définie dans la revendication 1 pour la préparation d'une composition, notamment médicamenteuse, pour favoriser la reconstruction cellulaire et/ou la différentiation cellulaire.

Cette composition est destinée à être avant tout un dispositif médical, et plus particulièrement un objet de pansement. Par « sucre non métabolisable » au sens de la présente demande, on entend un sucre qui ne peut être en totalité utilisé comme source d'énergie par l'organisme humain.

En particulier la présente invention concerne l'utilisation d'une association telle que définie dans la revendication 1 pour la préparation d'une composition, notamment médicamenteuse pour la reconstruction tissulaire en médecine humaine ou vétérinaire, dont les exemples d'application sont les suivants :
- injection pour combler les rides ou les déficits de tissu dermique ou sous-jacent à la peau dans un but de correction esthétique,
- Reconstruction du tissu nerveux : trauma de moelle épinière,
- Reconstruction osseuse : déficit de tissu osseux à la suite d'interventions ou de traumas,
- Reconstruction du cartilage : déficit associé à des pathologies dégénératives ou à des interventions chirurgicales.
- Traitement des brûlures
- Cicatrisations en général, avantageusement prévention et traitement des escarres.

La présente invention concerne également l'utilisation d'une association telle que définie dans la revendication 1 pour la préparation d'une composition, notamment médicamenteuse pour favoriser la différenciation cellulaire en médecine ou en biologie expérimentale :
- Traitement de tumeurs solides,
- Traitement des fonds de boîtes de culture cellulaire dans le but de favoriser la fixation, la différenciation, la migration et le développement de cellules en remplacement des matrices utilisées généralement dans le même but mais beaucoup plus coûteuses à l'emploi et moins polyvalentes, telles que le collagène, la fibronectine, la poly lysine, la laminine ou encore différentes associations commercialisées de ces substances.
- Cicatrisation, dont traitement ou prévention des escarres.

Le sucre non métabolisable est choisi dans le groupe formé par le xylose ou l'arabinose, de façon avantageuse, il s'agira du xylose qui est couramment utilisé dans l'alimentation humaine en tant que succédané du sucre et qui a fait la preuve de son innocuité.

L'absorbant est un polymère choisi parmi le polymère acrylique et le polymère carboxyvinylique. Il peut en particulier être choisi parmi les polyacrylates, les polyméthacrylates, et le polymère carboxypolyvinylique.

De façon avantageuse, il s'agit d'un polymère acrylique.

En particulier il est choisi parmi le polymère carboxypolyvinylique (carbomer), l'acide polyacrylique, le polyméthylmetacrylate, et l'acide polyacrylamide coacrylique. De façon avantageuse, il est choisi parmi le polymère carboxypolyvinylique (carbomer) et l'acide polyacrylique.

De façon encore plus avantageuse, il s'agit du polymère carboxypolyvinylique (carbomer).

De manière préférée, ladite composition se présente sous la forme d'une poudre. Cette formulation sous forme de poudre présente ainsi l'avantage de permettre une application facile et moins douloureuse que les applications de l'art antérieur. Ladite composition sous forme de poudre présente également l'avantage d'être fortement antiseptique de par les effets conjugués de la suppression de l'eau nécessaire à la survie bactérienne, l'absorption régulière des exsudats et la pression osmotique intense réalisée.

De préférence, les compositions comprendront entre 50 et 95 %, et de façon encore plus préférée entre 80 et 95 %, en masse, du sucre non métabolisable choisi parmi le xylose ou l'arabinose et entre 5 et 50 %, et de façon encore plus préférée entre 5 et 20 % en masse de l'absorbant.

Cette composition peut encore comprendre un agent choisi parmi les antibiotiques, les antiseptiques, les corticoïdes...

Il a en outre été établi que l'efficacité de la composition reste constante que l'absorbant et le sucre non métabolisable soient administrés simultanément, séparément ou de manière étalée dans le temps.

Les exemples suivants sont donnés à titre indicatif, non limitatif.

### Exemple 1 : Mise en évidence de l'effet de l'association de polymère carboxypolyvinylique (carbomer 914) et de xylose sur la migration, la fixation et la différenciation cellulaire de mélanocytes provenant d'une lignée continue.

On réalise un revêtement d'une boîte de culture à 6 puits avec un gel aqueux à partir d'un mélange de carbomer 914 et de xylose dans les proportions 1-9 (p/p)auquel on ajoute de l'eau distillée de façon à obtenir une concentration de 1% (p/p). On neutralise le gel formé à pH 7,00 par une solution d'hydroxyde de sodium N et on stérilise par autoclavage 20 minutes à 120°C.
Le gel stérile obtenu est dilué au 1/3 de façon stérile par du tampon PBS auquel on a ajouté 34 µg/ml de gentamycine. On place 1 ml de ce gel dilué dans chaque puits et on laisse sécher une nuit sous flux d'air laminaire stérile.

On ensemence ensuite par une suspension contenant 50000 cellules de mélanocytes de la lignée M4Beu (Tumourigenic phénotypes of human melanoma cell lines in nude mice determine by an active antitumour mechanism ; R. Jacubovich, H. Cabrillat, D. Gerlier, M. Bailly & J.F. Doré ; Br. J. Cancer (1985), 51, 335-345) dans 3 ml de milieu de culture MEM contenant 10% de sérum de veau, 1% de solution de vitamines (ref. Sigma M 6895), 1% de solution de pyruvate de sodium (ref. Sigma S 8636), 1% de solution d'acides aminés (ref.M 7145) et 50 µg/ml de gentamycine.

Les cultures sont mises à l'étuve à 37°C et 5% de CO2. On observe ensuite leur développement.

Des témoins sont réalisés sans revêtement du fond des puits.

On constate une forte différenciation cellulaire pour le mélange polymère carboxypolyvinylique et xylose associée à la formation d'amas cellulaires évoquant des formations tissulaires.

On constate aussi une assez bonne différenciation avec l'alginate de sodium et la carboxyméthyl cellulose seuls et en association avec le xylose. L'arabinose utilisé à la place du xylose donne aussi de bons résultats.

Ces résultats visibles par la morphologie cellulaire l'est aussi par la synthèse de mélanine réalisée par les mélanocytes qui représente un bon marqueur de différenciation.

### Exemple 2 : Mise en évidence de l'effet de l'association de polyacrylate et de xylose sur le traitement des escarres.

Des tests ont été réalisés sur 20 patients, 4 présentant des escarres au talon, les 16 autres des escarres sacrées.

Les escarres au talon mesuraient de 1 à 3 cm de diamètre au début du traitement. Les escarres sacrées de 4 à 12 cm. Deux d'entre elles laissaient apparaître le sacrum au fond de la lésion. Toutes ces escarres étaient fortement exsudatives.

Le traitement a consisté à appliquer une composition sous une forme poudre comprenant, en masse, 90 % de xylose et 10 % de polyacrylate, matin et soir de façon à recouvrir toute la surface intérieure de la lésion par une couche de poudre de 2 mm environ.

Dans tous les cas sans exception, on a pu constater une réduction du diamètre des escarres de 75 à 100 % et une disparition totale de la douleur en une semaine de traitement.

Les escarres au talon on disparu totalement en dix jours en laissant simplement une discrète inflammation sans douleur ni démangeaison.

Les 11 escarres sacrées les moins profondes possédaient également un diamètre compris entre 4 et 6 cm. Celles-ci se sont comblées pratiquement entièrement en deux semaines en laissant une simple dépression discrètement inflammatoire pour 3 d'entre elles ou même sans aucune inflammation pour les 8 autres.

Les 5 escarres les plus graves n'ont pas disparu entièrement mais l'épithélialisation a été complète à l'intérieur de la lésion en deux semaines. Le résultat final s'est présenté comme une perte de manière très inférieure à la perte de matière observée au début du traitement, sous forme d'une dépression plus ou moins régulière et plus ou moins accentuée. On a constaté une réduction du diamètre de la lésion de 75 à 85 %.

## Revendications

1. Utilisation d'une association comprenant un absorbant en tant que seul absorbant de la composition, choisi dans le groupe formé par un polymère acrylique et le polymère carboxyvinylique et au moins un sucre non métabolisable choisi parmi le xylose ou l'arabinose pour la préparation d'une composition, notamment médicamenteuse, pour favoriser la reconstruction cellulaire et/ou la différenciation cellulaire.

2. Utilisation selon la revendication 1 **caractérisé en ce que** la composition se trouve sous une forme injectable pour combler les rides ou les déficits de tissu dermique ou sous-jacent à la peau dans un but de correction esthétique.

3. Utilisation selon la revendication 1 pour la préparation d'une composition médicamenteuse, pour la reconstruction du tissu nerveux en particulier suite à un trauma de moelle épinière, pour la reconstruction osseuse, en particulier suite à un déficit de tissu osseux à la suite d'interventions ou de traumas, pour la reconstruction du cartilage en particulier suite à un déficit associé à des pathologies dégénératives ou à des interventions chirurgicales.

4. Utilisation selon la revendication 1 pour la préparation d'une composition médicamenteuse, pour le traitement des brûlures et la cicatrisation.

5. Utilisation selon la revendication 4 pour la préparation d'une composition médicamenteuse, pour prévenir ou soulager les escarres.

6. Utilisation selon la revendication 1 pour la préparation d'une composition médicamenteuse, pour le traitement de tumeurs solides.

7. Utilisation selon la revendication 1 pour la préparation d'une composition médicamenteuse, pour le traitement des fonds de boîtes de culture cellulaire dans le but de favoriser la fixation, la différenciation, la migration et le développement de cellules.

8. Utilisation selon l'une quelconque des revendications 1 à 7 telle que le sucre non métabolisable est le xylose.

9. Utilisation selon l'une quelconque des revendications 1 à 8 telle que l'absorbant est un polyacrylate, un polyméthacrylate, ou le polymère carboxyvinylique.

10. Utilisation selon la revendication 9 telle que l'absorbant est l'acide polyacrylique ou le polymère carboxyvinylique.

11. Utilisation selon la revendication 10 telle que l'absorbant est le polymère carboxyvinylique

12. Utilisation selon l'une des revendications 1, 3 à 11 telle que la composition se présente sous la forme d'une poudre.

13. Utilisation selon l'une des revendications précédentes telle que la composition comprend au moins un agent choisi dans le groupe formé par les antibiotiques, antiseptiques, corticoïdes.

14. Utilisation selon l'une des revendications précédentes telle que l'absorbant et le sucre non métabolisable sont administrés simultanément, séparément ou de manière étalée dans le temps.

15. Utilisation selon l'une des revendications précédentes telle que la composition comprend entre 50 et 95 % du sucre non métabolisable choisi parmi le xylose ou l'arabinose et entre 5 et 50 % en masse de l'absorbant.

16. Utilisation selon l'une des revendications précédentes telle que la composition comprend entre 80 et 95 % du sucre non métabolisable choisi parmi le xylose ou l'arabinose et entre 5 et 20 % en masse de l'absorbant.

## Claims

1. Use of a combination, comprising an absorbent as sole absorbent of the composition, selected from the group formed by an acrylic polymer and carboxyvinyl polymer and at least one nonmetabolizable sugar selected from xylose or arabinose, for preparing a composition, in particular a medicinal composition, for promoting cellular reconstruction and/or cellular differentiation.

2. Use according to Claim 1, **characterized in that** the composition is present in an injectable form for filing wrinkles or deficits of dermal tissue or tissue underlying the skin, for the purpose of esthetic correction.

3. Use according to Claim 1 for preparing a medicinal composition, for reconstructing nervous tissue, in particular following spinal cord trauma, for reconstructing bone, in particular following a deficit of bone tissue following interventions or traumas, or for reconstructing cartilage, in particular following a deficit associated with degenerative diseases or surgical interventions.

4. Use according to Claim 1 for preparing a medicinal composition, for treating burns and for healing.

5. Use according to Claim 4 for preparing a medicinal composition, for preventing or alleviating bedsores.

6. Use according to Claim 1 for preparing a medicinal composition, for treating solid tumors.

7. Use according to Claim 1 for preparing a medicinal composition, for treating the bottom of cell culture dishes with the aim of promoting the attachment, differentiation, migration and growth of cells.

8. Use according to any one of Claims 1 to 7, **characterized in that** the nonmetabolizable sugar is xylose.

9. Use according to one of Claims 1 to 8, **characterized in that** the absorbent is a polyacrylate, a polymethacrylate or carboxyvinyl polymer.

10. Use according to Claim 9, **characterized in that** the absorbent is polyacrylic acid or carboxyvinyl polymer.

11. Use according to Claim 10, **characterized in that** the absorbent is carboxyvinyl polymer.

12. Use according to one of Claims 1 and 3 to 11, **characterized in that** the composition is present in the form of a powder.

13. Use according to one of the preceding claims, **characterized in that** the composition comprises at least one agent which is selected from the group formed by antibiotics, antiseptics and corticosteroids.

14. Use according to one of the preceding claims, **characterized in that** the absorbent and the nonmetabolizable sugar are administered simultaneously, separately or such that the administrations are staggered over time.

15. Use according to one of the preceding claims, **characterized in that** the composition comprises between 50 and 95% of the nonmetabolizable sugar selected from xylose or arabinose and between 5 and 50%, by weight, of the absorbent.

16. Use according to one of the preceding claims, **characterized in that** the composition comprises between 80 and 95% of the nonmetabolizable sugar selected from xylose or arabinose and between 5 and 20%, by weight, of the absorbent.

## Patentansprüche

1. Verwendung einer Assoziation, die ein Absorbens als einziges Absorbens der Zusammensetzung, ausgewählt aus der aus einem Vinylpolymer und dem Carboxyvinylpolymer gebildeten Gruppe, und mindestens einen nicht metabolisierbaren Zucker umfasst, der aus Xylose oder Arabinose ausgewählt ist, für die Herstellung einer insbesondere medikamentösen Zusammensetzung, um die Zellwiederherstellung und/oder die Zelldifferenzierung zu begünstigen.

2. Verwendung nach Anspruch 1, **dadurch gekennzeichnet, dass** die Zusammensetzung in injizierbarer Form vorliegt, um Falten oder Defizite von dermischem oder unter der Haut liegendem Gewebe mit dem Ziel einer ästhetischen Korrektur aufzufüllen.

3. Verwendung nach Anspruch 1 für die Herstellung einer medikamentösen Zusammensetzung für die Wiederherstellung von Nervengewebe insbesondere nach Rückenmarktrauma, für die Wiederherstellung von Knochen insbesondere nach einem Defizit von Knochengewebe infolge von Eingriffen oder Traumata, für die Wiederherstellung von Knorpel insbesondere nach einem Defizit, das mit degenerativen Krankheiten oder chirurgischen Eingriffen verbunden ist.

4. Verwendung nach Anspruch 1 für die Herstellung einer medikamentösen Zusammensetzung für die Behandlung von Verbrennungen und Narbenbildungen.

5. Verwendung nach Anspruch 4 für die Herstellung einer medikamentösen Zusammensetzung, um Schorf zu verhüten oder zu verbessern.

6. Verwendung nach Anspruch 1 für die Herstellung einer medikamentösen Zusammensetzung für die Behandlung von festen Tumoren.

7. Verwendung nach Anspruch 1 für die Herstellung einer medikamentösen Zusammensetzung für die Behandlung von Böden von Schalen für die Zellkultur mit dem Ziel, die Fixierung, Differenzierung, Migration und Entwicklung von Zellen zu begünstigen.

8. Verwendung nach irgendeinem der Ansprüche 1 bis 7, derart, dass der nicht metabolisierbare Zucker Xylose ist.

9. Verwendung nach irgendeinem der Ansprüche 1 bis 8, derart, dass das Absorbens ein Polyacrylat, ein Polymethacrylat oder das Carboxyvinylpolymer ist.

10. Verwendung nach Anspruch 9, derart, dass das Absorbens Polyacrylsäure, oder das Carboxyvinylpolymer ist.

11. Verwendung nach Anspruch 10, derart, dass das Absorbens das Carboxyvinylpolymer ist.

12. Verwendung nach einem der Ansprüche 1, 3 bis 11, derart, dass die Zusammensetzung in Form eines Pulvers vorliegt.

13. Verwendung nach einem der vorangehenden Ansprüche, derart, dass die Zusammensetzung mindestens ein Mittel umfasst, das ausgewählt ist aus der Gruppe, die aus Antibiotika, Antiseptika, Corticoiden gebildet ist.

14. Verwendung nach einem der vorangehenden Ansprüche, derart, dass das Absorbens und der nicht metabolisierbare Zucker simultan, getrennt oder auf zeitlich gestaffelte Weise verabreicht werden.

15. Verwendung nach einem der vorangehenden Ansprüche, derart, dass die Zusammensetzung zwischen 50 und 95 Massen-% nicht metabolisierbaren Zucker, der aus Xylose und Arabinose ausgewählt ist, und zwischen 5 und 50 Massen-% des Absorbens umfasst.

16. Verwendung nach einem der vorangehenden Ansprüche, derart, dass die Zusammensetzung zwischen 80 und 95 Massen-% nicht metabolisierbaren Zucker, der aus Xylose und Arabinose ausgewählt ist, und zwischen 5 und 20 Massen-% des Absorbens umfasst.
